# EUROPEAN PATENT APPLICATION

(11) **EP 3 323 392 A1**
(43) Date of publication of application: **23.05.2018**
(21) Application number: 16824717.9
(22) Date of filing: 13.07.2016
(51) Int. Cl.: A61F 5/455, A61F 5/44

(54) **SMART MENSTRUAL CUP AND METHOD FOR MEASURING MENSTRUAL BLOOD USING SMART MENSTRUAL CUP**

(30) Priority: 13.07.2015 KR 20150098986; 13.07.2015 KR 20150099024
(71) Applicant: Loon Lab Inc., Seongnam-si, Gyeonggi-do 13466 (KR)
(72) Inventor: HWANG, Ryong, Cheonan-si Chungcheongnam-do 31207 (KR)
(74) Representative: Groth & Co. KB
(86) International application number: PCT/KR2016/007597
(87) International publication number: WO 2017/010800

(57) **Abstract**

An embodiment of the present invention provides a smart menstrual cup comprising: a storage unit, a side of which is open so as to receive menstrual blood in at least one inner space thereof; a measuring unit for measuring menstrual blood received in the storage unit; and a control unit for generating a signal on the basis of measurement information from the measuring unit or processing information obtained on the basis of the measurement information.

## Description

### TECHNICAL FIELD

Embodiments of the present invention relate to a smart menstrual cup and a method of measuring menstrual blood by using the smart menstrual cup.

### BACKGROUND ART

When women menstruate, they use menstrual blood leakage prevention tools, such as sanitary pads, menstrual cups, and tampons, to prevent leakage of menstrual blood from the vagina, for both hygiene and comfort.

From among such tools, a sanitary pad is uncomfortable to wear and may involve external leakage of menstrual blood. A tampon may sometimes cause a toxic shock syndrome. A menstrual cup is inserted into the vagina and thus may prevent external leakage of menstrual blood and cause less inconvenience to a user's life. However, as in the cases of other menstrual blood leakage prevention tools, it is difficult to figure out an amount of menstruation before the menstrual cup is removed.

Also, if color of menstrual blood may be correctly determined, information regarding the state of health of a woman, etc. may be checked. However, when conventional menstrual blood leakage prevention tools are used, it is difficult to determine the color of menstrual blood.

If an amount of menstruation may be figured out before a menstrual blood leakage prevention tool is removed, it may become more convenient to manage the menstrual blood leakage prevention tool. Also, if the color of menstrual blood may be correctly determined, a state of health may be checked.

Information disclosed in this Background section was already known to the inventors before achieving the inventive concept or is technical information acquired in the process of achieving the inventive concept. Therefore, it may contain information that does not form the prior art that is already known to the public in this country.

### DETAILED DESCRIPTION OF THE INVENTION

### TECHNICAL PROBLEM

Embodiments of the present invention provide a smart menstrual cup that allows an amount of menstruation, a color of menstrual blood, a menstrual cycle, or the like to be determined even before the menstrual cup is removed.

Embodiments of the present invention provide a smart menstrual cup capable of providing information regarding a management period of the menstrual cup by providing a method of determining an amount of menstruation even before removing a menstrual blood leakage prevention tool.

Embodiments of the present invention provide a smart menstrual cup capable of providing information regarding a state of health of a user, etc. by providing a method of correctly determining a color of menstrual blood.

Embodiments of the present invention provide a smart menstrual cup that allows a user to figure out a management period of the menstrual cup by providing an appropriate sense signal to the user according to an amount, a weight, or the like of menstrual blood stored in the menstrual cup.

### TECHNICAL SOLUTION

According to an embodiment of the present invention, there is provided a smart menstrual cup including: a storage unit, a side of which is open to receive menstrual blood in at least one inner space thereof; a measuring unit for measuring the menstrual blood received in the storage unit; and a control unit for generating a signal based on measurement information from the measuring unit or processing information obtained based on the measurement information.

The smart menstrual cup may further include a partitioning unit between the storage unit and the measuring unit, the partitioning unit preventing the menstrual blood received in the storage unit from leaking into the measuring unit or the control unit.

The control unit may include a first substrate, a battery unit on a surface of the first substrate, a second substrate on a surface of the battery unit, and a connecting unit for electrically connecting the first substrate and the second substrate, and the measuring unit may be located on the first substrate.

The smart menstrual cup may further include a communication unit electrically connected to the control unit and transmitting the signal generated in the control unit to a device.

The measuring unit may include a color sensor, and the control unit may generate a data signal including color information of menstrual blood measured via the color sensor, or a data signal including health information according to a menstrual blood color obtained from the color information.

The measuring unit may include a weight sensor, and the control unit may generate a data signal including weight information of menstrual blood measured via the weight sensor, or an alarm signal according to whether a weight of the menstrual blood is greater than a predetermined reference weight.

The measuring unit may be a sensor formed on an inner side of the storage unit where the menstrual blood is received, and the control unit may generate an alarm signal according to whether a level, an amount, or a volume of menstrual blood measured via the measuring unit is greater than a reference value.

The smart menstrual cup may further include a sense signal generator for generating a sense signal capable of being sensed via at least one of five human senses, based on the signal generated in the control unit.

In the storage unit, a circumference of a location spaced apart by a first distance from the open side of the storage unit may be greater than a circumference of a location spaced apart by a second distance greater than the first distance from the open side of the storage unit.

In the storage unit, an area of a cross-section at a location spaced apart by a first distance from the open side of the storage unit may be greater than an area of a cross-section at a location spaced apart by a second distance greater than the first distance from the open side of the storage unit.

According to another embodiment of the present invention, there is provided a method of measuring menstrual blood by using a smart menstrual cup, the method including: receiving menstrual blood via a storage unit of the smart menstrual cup; measuring the menstrual blood stored in the storage unit via a measuring unit of the smart menstrual cup; and generating a signal, based on measurement information from the measuring unit or processing information obtained based on the measurement information, via a control unit of the smart menstrual cup.

The measuring may include measuring a color of the menstrual blood by using a color sensor, and the generating may include generating a data signal including color measurement information of the menstrual blood, or a data signal including health information according to a menstrual blood color obtained from the color information.

The measuring may include measuring a weight of the menstrual blood by using a weight sensor, and the generating may include generating a data signal including weight measurement information of the menstrual blood, or an alarm signal according to whether a weight of the menstrual blood is greater than a predetermined reference weight.

The measuring may include measuring a level, an amount, or a volume of the menstrual blood by using a sensor located on an inner side of the storage unit, and the generating may include generating a data signal including measurement information regarding the level, the amount, or the volume of the menstrual blood, or an alarm signal according to whether the amount of the menstrual blood is greater than a reference value.

The method may further include, after the generating, generating a sense signal capable of being sensed via at least one five human senses, based on the signal generated in the control unit, via a sense signal generator of the smart menstrual cup.

Additional aspects will be set forth in part in the description which follows and, in part, will be apparent from the description, or may be learned by practice of the presented embodiments.

### ADVANTAGEOUS EFFECTS OF THE INVENTION

As described above, according to the present invention, a smart menstrual cup that allows an amount of menstruation, a color of menstrual blood, a menstrual cycle, or the like to be figured out even before the menstrual cup is removed may be provided.

In addition, according to the present invention, a smart menstrual cup capable of providing information regarding a management period of the menstrual cup may be provided by providing a method of figuring out an amount of menstruation even before removing a menstrual blood leakage prevention tool.

In addition, according to the present invention, a smart menstrual cup capable of providing information regarding a state of health of a user, etc. may be provided by providing a method of correctly determining a color of menstrual blood.

In addition, according to the present invention, a smart menstrual cup that allows a user to figure out a management period of the menstrual cup may be provided by providing an appropriate sense signal to the user according to an amount, a weight, or the like of menstrual blood stored in the menstrual cup.

### DESCRIPTION OF THE DRAWINGS

FIG. 1 is a perspective view of a smart menstrual cup according to an embodiment of the present invention.
FIG. 2 is a cross-sectional view of a smart menstrual cup according to an embodiment of the present invention.
FIG. 3 is a diagram of a smart menstrual blood management system according to an embodiment of the present invention.
FIGS. 4 to 6 are diagrams of elements of a smart menstrual cup according to an embodiment of the present invention.
FIG. 7 is a diagram of a smart menstrual cup according to another embodiment of the present invention.
FIG. 8 is a diagram of a control unit and other elements of a smart menstrual cup according to another embodiment of the present invention.
FIG. 9 is a diagram of a smart menstrual cup according to another embodiment of the present invention.

### BEST MODE

As the present invention allows for various changes and numerous embodiments, certain embodiments will be illustrated in the drawings and described in detail in the written description. Effects and features of the present invention and methods of accomplishing the same may be understood more readily by reference to the following detailed description of embodiments and the accompanying drawings. The present invention may, however, be embodied in many different forms and should not be construed as being limited to the embodiments set forth herein. While such terms as "first" and "second" may be used to describe various elements, such elements must not be limited to the above terms. The above terms are used only to distinguish one element from another. The singular forms "a," "an," and "the" used herein are intended to include the plural forms as well, unless the context clearly indicates otherwise. It will be understood that the terms such as "include," "comprise," and "have" used herein specify the presence of stated features or elements, but do not preclude the presence or addition of one or more other features or elements. Sizes of elements in the drawings may be exaggerated for convenience of explanation. In other words, since sizes and thicknesses of elements in the drawings are arbitrarily illustrated for convenience of explanation, the present invention is not limited thereto.

Hereinafter, embodiments of the present invention will be described in detail with reference to the accompanying drawings. Like reference numerals in the drawings denote like elements, and repeated description thereof will be omitted.

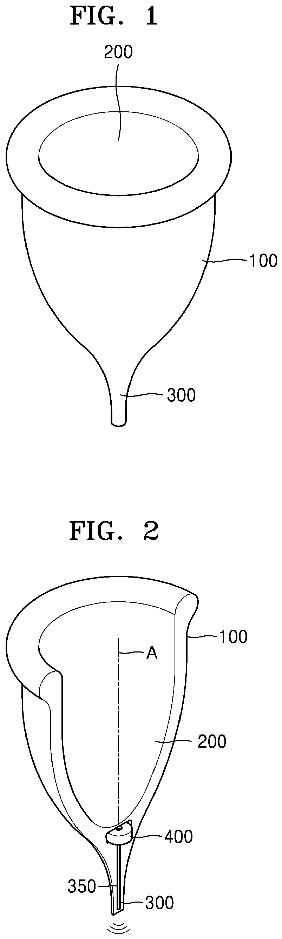

FIG. 1 is a perspective view of a smart menstrual cup according to an embodiment of the present invention. FIG. 2 is a cross-sectional view of a smart menstrual cup according to an embodiment of the present invention.

Referring to FIGS. 1 and 2, a smart menstrual cup 100 according to an embodiment of the present invention may include a storage unit 200 and a grip unit 300. Also, the smart menstrual cup 100 may further include a communication unit 350 and a control unit 400.

The smart menstrual cup 100 may have a cup shape and may include a space having one side (that may be hereinafter referred to as an upper side) that is open and capable of receiving menstrual blood. The smart menstrual cup 100 may include the storage unit 200 having the upper side open to receive menstrual blood. The smart menstrual cup 100 may be manufactured such that an upper diameter decreases downwards. Alternatively, in the smart menstrual cup 100, a circumference of the storage unit 200 at a location spaced apart by a first distance from the open one side may be greater than that of the storage unit 200 at a location spaced apart by a second distance greater than the first distance from the open one side. That is, for correct insertion and fixing, in at least a partial region of the smart menstrual cup 100, a circumference at a portion close to an opening may be greater than that at a portion far from the opening. As another example, with respect to a cross-section perpendicular to an axis A of FIG. 2, an area of the cross-section at a location spaced apart by a first distance from the open one side of the smart menstrual cup 100 may be greater than that of the cross-section at a location spaced apart by a second distance greater than the first distance from the open one side. However, a form of the smart menstrual cup 100 according to the present invention is not limited thereto, and the smart menstrual cup 100 may have various forms. Hereinafter, a form in which a circumference at an upper portion is greater than that at a lower portion will be described as an example.

The smart menstrual cup 100 may include an elastic material, such as rubber or silicone, bending easily, so that the smart menstrual cup 100 may be inserted into the vagina while being bent. However, a material of the smart menstrual cup 100 is not limited to rubber or silicone, and only one portion of the smart menstrual cup 100 may include an elastic material and another portion thereof may include a non-elastic material.

A user of the smart menstrual cup 100 may insert and fix the smart menstrual cup 100 into the vagina, and menstrual blood may be stored in the storage unit 200 via an opening at the upper side of the smart menstrual cup 100 to prevent external leakage of menstrual blood from the user's vagina.

The smart menstrual cup 100 may further include, at the bottom, the grip unit 300 for allowing the user to carry, insert, or remove the smart menstrual cup 100 more easily. The grip unit 300 may protrude from the bottom of the smart menstrual cup 100 in an opposite direction to the opening of the storage unit 200. The grip unit 300 may have various forms, such as a pillar form or a ring form, making it easy to hold a smart menstrual cup with a hand or deal with a smart menstrual cup by using a hand. However, a case in which the grip unit 300 has a pillar shape will hereinafter be described as an example.

The communication unit 350 may be electrically connected to the control unit 400 and may externally transmit a signal generated in the control unit 400. The communication unit 350 may transmit the signal to an external electronic device separated from the smart menstrual cup 100. For example, the communication unit 350 may transmit a signal generated in the control unit 400 to a device (for example, a smartphone) of the user of the smart menstrual cup 100.

The communication unit 350 may stretch through the inside of the grip unit 300 of the smart menstrual cup 100. In this regard, the communication unit 350 may extend to the bottom of the smart menstrual cup 100 through the inside of the grip unit 300. The reason for this is that, if the communication unit 350 is only in the vagina, it may be difficult to wirelessly communicate with an external electronic device. However, this is just an embodiment, and the communication unit 350 may be included in the control unit 400 or may be disposed at other various locations of the smart menstrual cup 100. In addition, the communication unit 350 may detect when the smart menstrual cup 100 is outside the body, and only at this time, may transmit a communication signal to the device of the user.

The communication unit 350 may have a linear form, a curved form, or the like, and may have the form of an antenna including a material such as metal. Also, the communication unit 350 may include an element for communicating with the device of the user by using means of communication such as Bluetooth, infrared rays, Zigbee, Wi-Fi, etc.

The control unit 400 may generate a signal based on information related to menstrual blood stored in the smart menstrual cup 100. The control unit 400 may generate various signals based on various pieces of information related to the stored menstrual blood. The control unit 400 may include a computer-readable recording medium in which a computer program is stored. In this regard, the computer program may be an implementation of an algorithm for controlling various elements of the smart menstrual cup 100. The control unit 400 may include various electronic components required to drive the computer-readable recording medium. The control unit 400 may include one or more substrates on which the electronic components are disposed.

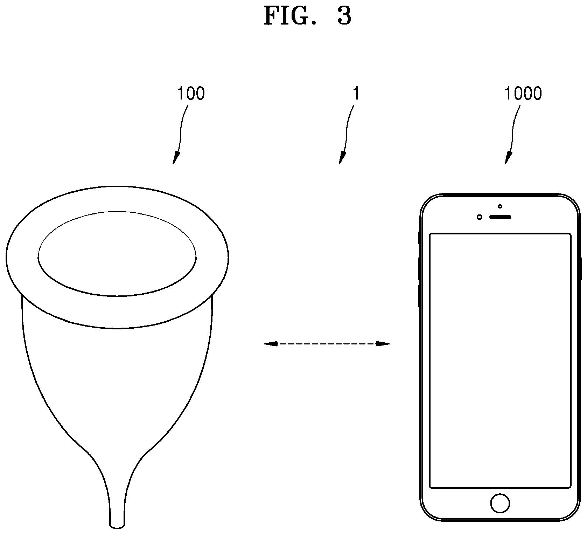

FIG. 3 is a diagram of a smart menstrual blood management system according to an embodiment of the present invention.

Referring to FIG. 3, the smart menstrual cup 100 according to an embodiment of the present invention may operate as one element of a smart menstrual blood management system 1. In this regard, various signals generated in the smart menstrual cup 100 may be transmitted to an external electronic device 1000 via a communication network. Selectively, the smart menstrual cup 100 may receive a signal from the external electronic device 1000 via the communication network. That is, the smart menstrual cup 100 may generate a signal including information regarding an amount of menstrual blood received in the smart menstrual cup 100 or a color of menstrual blood by measuring or analyzing menstrual blood received in the smart menstrual cup 100, and may transmit the signal to the external electronic device 1000. Alternatively, the smart menstrual cup 100 may generate a signal including various pieces of body information that may be obtained from an amount or a color of menstrual blood or raw data for obtaining body information, and may transmit the signal to the external electronic device 1000.

### MODE OF THE INVENTION

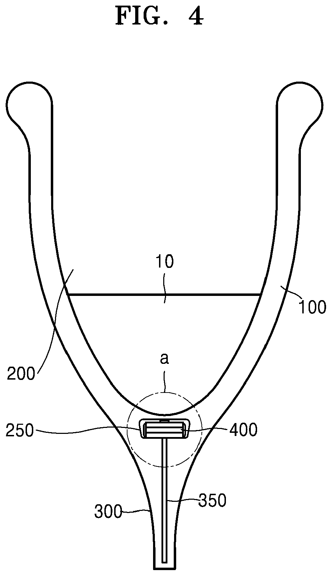

FIGS. 4 to 6 are diagrams of elements of a smart menstrual cup according to an embodiment of the present invention.

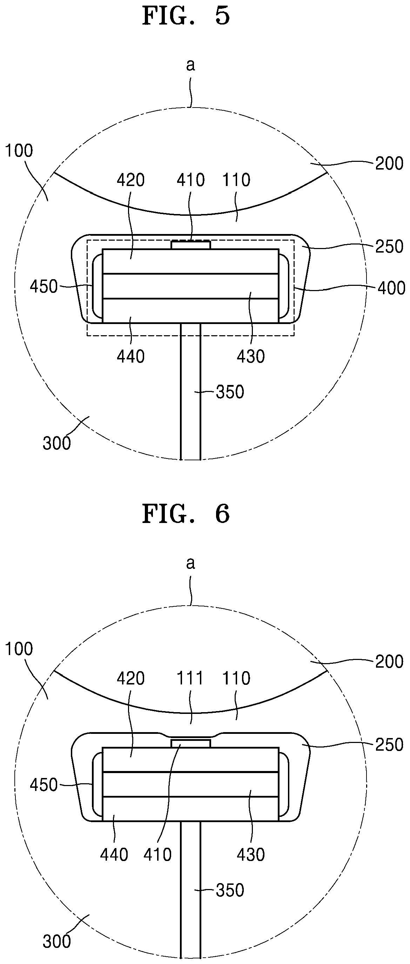

Referring to FIG. 4, the smart menstrual cup 100 according to an embodiment of the present invention may include the storage unit 200, the grip unit 300, the communication unit 350, and the control unit 400. Menstrual blood 10 may be received in the storage unit 200. In this regard, various elements of the smart menstrual cup 100 may be disposed at a lower end of the storage unit 200 and a peripheral portion a of the control unit 400. Example forms in which the various elements are disposed at the lower end of the storage unit 200 and the peripheral portion a of the control unit 400 will be described with reference to FIGS. 5 and 6.

Referring to FIG. 5, the smart menstrual cup 100 may further include a partitioning unit 110 between the storage unit 200 and the control unit 400. Also, the smart menstrual cup 100 may include a measuring unit 410 for measuring characteristics of menstrual blood. The control unit 400 may include a first substrate 420, a battery unit 430, a second substrate 440, and a connecting unit 450.

The partitioning unit 110 may prevent menstrual blood received in the storage unit 200 from flowing into the control unit 400 or another region of the smart menstrual cup 100. In this regard, the partitioning unit 110 and the other region of the smart menstrual cup 100 may include different materials from each other. Alternatively, the partitioning unit 110 and the other region of the smart menstrual cup 100 may include the same material as each other, and the partitioning unit 110 may refer to a region simply specified by a location thereof.

The measuring unit 410 may be formed on the control unit 400. For example, the measuring unit 410 may be formed on the first substrate 420 disposed at the top of the control unit 400. The bottom of the first substrate 420 may be electrically connected to a surface of the battery unit 430. Alternatively, the measuring unit 410 may be included in the control unit 400. Also, the smart menstrual cup 100 may include an element arrangement space 250 which is a space for the entire control unit 400 or the measuring unit 410.

Although FIG. 5 illustrates the measuring unit 410 that is smaller than the control unit 400, this is just an example, and a size of the measuring unit 410 may be similar to or greater than that of the control unit 400. Also, although FIG. 5 illustrates one measuring unit 410 that is formed on the control unit 400, this is just an example, and two or more measuring units 410 may be formed on the control unit 400. In this case, at least some of the two or more measuring units 410 may perform a different role from the other measuring units 410. For example, one measuring unit 410 may measure weight of menstrual blood received in the storage unit 200, and another measuring unit 410 may measure a color of the menstrual blood received in the storage unit 200.

The battery unit 430 may supply power for driving the control unit 400, the measuring unit 410 and/or the communication unit 350. The battery unit 430 may be various types of batteries. For example, a surface of the battery unit 430 may be a positive or negative terminal, and another surface thereof may be a terminal having different polarity from the surface. The battery unit 430 may be a battery in the form of a flat cylinder. In this regard, a surface of the battery unit 430 may be electrically connected to the first substrate 420, and another surface of the battery unit 430 may be electrically connected to the second substrate 440.

The second substrate 440 may be electrically connected to the communication unit 350. The second substrate 440 may be electrically connected to the first substrate 420 via the connecting unit 450. The connecting unit 450 may be a conductor. The connecting unit 450 may be a film-type conductor, electric wiring, or the like. Via the structure described above, the measuring unit 410 and the communication unit 350 may receive power from the battery unit 430.

Selectively, the measuring unit 410 may include a weight sensor. The measuring unit 410 including the weight sensor may sense weight (or force) applied from a direction of the partitioning unit 110. In detail, referring to FIG. 6, as menstrual blood is received in the storage unit 200, the partitioning unit 110 bears weight of menstrual blood stored in the storage unit 200, and as a result, the partitioning unit 110 may move toward the element arrangement space 250. Accordingly, as the partitioning unit 110 moves toward the element arrangement space 250, a force pressing the measuring unit 410 may be generated. The weight sensor included in the measuring unit 410 may electrically or physically sense the force pressing the measuring unit 410.

The control unit 400 may generate a signal based on weight measured via the measuring unit 410. For example, the control unit 400 may generate a signal including information regarding a numerical value, a color, a geometrical figure, a sound, a degree of vibration, etc. changing in proportion to the weight measured via the measuring unit 410. The signal generated in the control unit 400 may be transmitted to an external electronic device via the communication unit 350. In this regard, the external electronic device may check information included in the received signal and output the information to a user. For example, the control unit 400 may generate a signal including numerical value information changing in proportion to weight measured via the measuring unit 410. In this case, the external electronic device may visually display the numerical value, and the user of the smart menstrual cup 100 may check an amount of menstrual blood currently received in the smart menstrual cup 100 by checking the numerical value. As another example, the control unit 400 may generate an alarm signal according to whether a weight measured via the measuring unit 410 is greater than a reference weight. In this case, when the external electronic device receives the alarm signal, the external electronic device may provide information that the weight of menstrual blood currently received in the smart menstrual cup 100 is greater than the reference weight, by providing auditory, visible, or haptic stimulation to the user of the smart menstrual cup 100.

When the partitioning unit 110 moves toward the element arrangement space 250 due to weight of menstrual blood, if an area of the measuring unit 410 is less than that of the partitioning unit 110, it may be difficult to accurately measure weight. Accordingly, in order to solve the above problem, a protrusion 111 of a size corresponding to a size of the measuring unit 410 itself or the weight sensor included in the measuring unit 410 may be provided at the bottom of the partitioning unit 110 of the smart menstrual cup 100. In detail, when an area of the measuring unit 410 itself or the weight sensor included in the measuring unit 410 is less than that of the partitioning unit 110, it may be difficult to correctly sense a change in weight according to a degree of movement of the partitioning unit 110. Accordingly, the smart menstrual cup 100 may include the protrusion 111 that is smaller in size than the measuring unit 410 itself or the weight sensor included in the measuring unit 410.

The second substrate 440 and/or the battery unit 430 may be fixed to the smart menstrual cup 100. In detail, the partitioning unit 110 may move downwards due to weight of the menstrual blood 10, and if the second substrate 440 and/or the battery unit 430 are not fixed to the smart menstrual cup 100, the measuring unit 410 may also move along with the second substrate 440 and/or the battery unit 430, and as a result, characteristics of menstrual blood to be measured via the measuring unit 410 may not be correctly measured. Accordingly, the bottom or a portion of the second substrate 440 and/or the battery unit 430 may be fixed to the smart menstrual cup 100.

Selectively, the measuring unit 410 may include a color sensor. The measuring unit 410 including the color sensor may identify color of menstrual blood received in the storage unit 200, based on light penetrating the partitioning unit 110. In this regard, the entire smart menstrual cup 100 or a portion of the smart menstrual cup 100 may include a material that may transmit light easily. Alternatively, the entire partitioning unit 110 or a portion of the partitioning unit 110 may include a material that may transmit light easily. For example, at least a portion of the smart menstrual cup 100 and/or the partitioning unit 110 may include a completely transparent material or a material that may transmit light of a predetermined level or greater.

The color sensor may be an RGB sensor capable of sensing red, green, and blue. However, a type of the color sensor is not limited thereto, and the color sensor may be various types of sensors capable of distinguishing light or color.

The control unit 400 may generate a signal based on a color measured via the measuring unit 410. For example, the control unit 400 may generate a signal including information regarding the color itself measured via the measuring unit 410. Alternatively, the control unit 400 may determine a state of health of the user of the smart menstrual cup 100, according to the color measured via the measuring unit 410, and may generate a signal including information regarding the determined state of health. The signal generated in the control unit 400 may be transmitted to an external electronic device via the communication unit 350. In this regard, the external electronic device may check and output information included in the received signal. For example, the control unit 400 may generate a signal including color information measured via the measuring unit 410. In this case, the external electronic device may visually display the color, and thus, the user of the smart menstrual cup 100 may check the color of menstrual blood currently received in the smart menstrual cup 100.

The measuring unit 410 including the color sensor may be fixed to the partitioning unit 110. In detail, the partitioning unit 110 may move due to pressure of menstrual blood applied to the partitioning unit 110. If the partitioning unit 110 and the measuring unit 410 are not fixed, a distance between the partitioning unit 110 and the measuring unit 410 may change, and thus, an error may occur in measurement of color via the color sensor included in the measuring unit 410. Accordingly, the measuring unit 410 may be fixed to the partitioning unit 110. Alternatively, an end of the first substrate 420 on which the measuring unit 410 is formed, or a portion thereof, may be fixed to the partitioning unit 110.

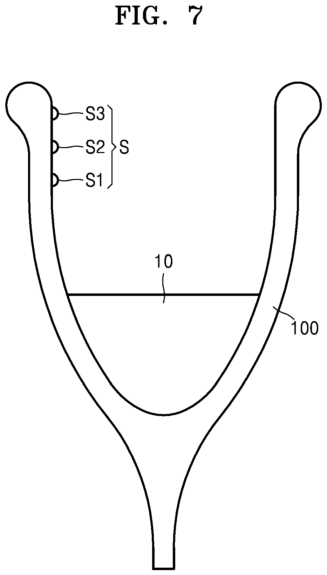

FIG. 7 is a diagram of a smart menstrual cup according to another embodiment of the present invention.

Referring to FIG. 7, the smart menstrual cup 100 may include a measuring sensor S in the storage unit 200 of the smart menstrual cup 100. In this regard, the measuring sensor S may be various types of sensors such as a contact sensor, a moisture sensor, a light sensor, or the like. The measuring sensor S may measure whether menstrual blood is received as high as a location of the measuring sensor S or the vicinity thereof. Afterwards, the measuring sensor S may transmit a measurement result to the control unit 400. Thus, the control unit 400 may check whether an amount or a level of menstrual blood received in the storage unit 200 is greater than a reference value, and may generate a signal accordingly. Alternatively, the control unit 400 may calculate a volume of received menstrual blood from a measured level of menstrual blood and a previously inputted inner product of a container of a menstrual cup, and the control unit 400 may generate a corresponding signal.

In this regard, the number of measuring sensors S in the storage unit 200 of the smart menstrual cup 100 may be two or more. For example, the smart menstrual cup 100 may include a first measuring sensor S1, a second measuring sensor S2, and a third measuring sensor S3 in the storage unit 200. In this case, the control unit 400 may generate an alarm signal to the user, based on a measurement result from each of the measuring sensors S. For example, when menstrual blood is identified only by the first measuring sensor S1, the control unit 400 may generate a weak warning signal. When menstrual blood is identified by the first measuring sensor S1 and the second measuring sensor S2, the control unit 400 may generate a strong warning signal. When menstrual blood is identified by all of the first to third measuring sensors S1 to S3, the control unit 400 may generate a danger signal. Alternatively, the smart menstrual cup 100 may check an amount or a level of menstrual blood received in the storage unit 200 by using a plurality of measuring sensors S disposed in the storage unit 200, and may calculate a volume of the received menstrual blood from the checked level and a previously inputted inner product of a container of the menstrual cup.

Alternatively, the measuring sensor S may be a sensor, such as a capacitance sensor, specifying electrical characteristics. A plurality of measuring sensors S may be conductors and may be disposed from an upper portion of the smart menstrual cup 100 to a lower portion of the smart menstrual cup 100, and the control unit 400 may measure a volume of menstrual blood by using electrical characteristics (for example, dielectric constant) measured from the plurality of measuring sensors S.

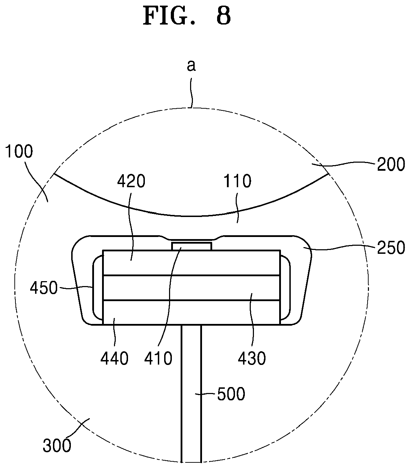

FIG. 8 is a diagram of a control unit and other elements of a smart menstrual cup according to another embodiment of the present invention.

Referring to FIG. 8, the smart menstrual cup 100 may further include a sense signal generator 500 generating a sense signal that may be sensed via at least one of the five human senses. The sense signal may be a haptic signal generating minute vibration, a signal generating a minute change in temperature, a signal generating a minute sound, or any other signal generating a phenomenon that may be sensed via at least one of the five human senses.

The control unit 400 may control the sense signal generator 500 to generate a sense signal in the sense signal generator 500 according to a result measured via the measuring unit 410. In this regard, the control unit 400 may generate a control signal for controlling the sense signal generator 500. For example, when it is determined based on a result of measurement via the measuring unit 410 that menstrual blood of a predetermined reference or greater is received in the smart menstrual cup 100, the control unit 400 may generate a control signal for generating a sense signal in the sense signal generator 500, based on the measurement result of the measuring unit 410. The sense signal generator 500 may generate a sense signal according to the control signal from the control unit 400. For example, the sense signal generator 500 may generate minute vibration that may be recognized by the user of the smart menstrual cup 100 but may not be sensed by other people around the user. Thus, the user of the smart menstrual cup 100 may check an amount of menstrual blood received in the smart menstrual cup 100 while the user maintains privacy.

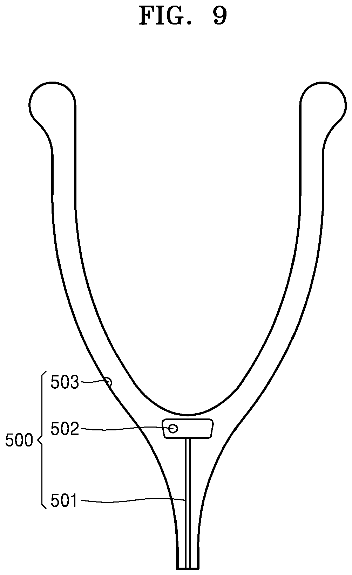

FIG. 9 is a diagram of a smart menstrual cup according to another embodiment of the present invention.

Referring to FIG. 9, the smart menstrual cup 100 may include the sense signal generator 500 in at least one of various locations. For example, the smart menstrual cup 100 may include a first sense signal generator 501 extending in the grip unit 300. As another example, the smart menstrual cup 100 may include a second sense signal generator 502 included in the control unit 400. As another example, the smart menstrual cup 100 may include a third sense signal generator 503 at the outside or inside of the storage unit 200. Alternatively, the smart menstrual cup 100 may include one or more sense signal generators 500 in at least one of the various locations. Although not illustrated, the smart menstrual cup 100 may include both of the communication unit 350 and the sense signal generator 500. In addition, the smart menstrual cup 100 may include a device including functions of at least two of the storage unit 200, the grip unit 300, the communication unit 350, the control unit 400, and/or the sense signal generator 500.

The particular implementations shown and described herein are illustrative examples of the invention and are not intended to otherwise limit the scope of the invention in any way. For the sake of brevity, description of conventional electronics, control systems, software development, and other functional aspects of the systems may be omitted. Furthermore, the connecting lines, or connectors shown in the various figures presented are intended to represent example functional relationships and/or physical or logical couplings between the various elements. It should be noted that many alternative or additional functional relationships, physical connections, or logical connections may be present in a practical device. Moreover, no item or component is essential to the practice of the invention unless the element is specifically described as "essential" or "critical".

Accordingly, the spirit of the present invention should not be limited to the embodiments described herein, and it is to be appreciated that not only the appended claims but also all the equivalents and changes within the claims are encompassed in the spirit of the present invention.

### INDUSTRIAL APPLICABILITY

The present invention relates to a smart menstrual cup that allows a management period of the menstrual cup to be determined by allowing an amount of menstruation to be determined even before removing a menstrual blood leakage prevention tool, and is capable of providing information regarding a state of health of a user, etc. by providing a method of correctly determining a color of menstrual blood, and a method of measuring menstrual blood by using the smart menstrual cup. The present invention may be used to manufacture a smart menstrual cup for providing a convenient method of use to the user and providing useful information.

## Claims

1. A smart menstrual cup comprising:
a storage unit, a side of which is open to receive menstrual blood in at least one inner space thereof;
a measuring unit for measuring the menstrual blood received in the storage unit; and
a control unit for generating a signal based on measurement information from the measuring unit or processing information obtained based on the measurement information.

2. The smart menstrual cup of claim 1, further comprising a partitioning unit between the storage unit and the measuring unit, the partitioning unit preventing the menstrual blood received in the storage unit from leaking into the measuring unit or the control unit.

3. The smart menstrual cup of claim 2, wherein the control unit comprises a first substrate, a battery unit on a surface of the first substrate, a second substrate on a surface of the battery unit, and a connecting unit for electrically connecting the first substrate and the second substrate, and
the measuring unit is located on the first substrate.

4. The smart menstrual cup of claim 1, further comprising a communication unit electrically connected to the control unit and transmitting the signal generated in the control unit to a device.

5. The smart menstrual cup of claim 1, wherein the measuring unit comprises a color sensor, and
the control unit generates a data signal comprising color information of menstrual blood measured via the color sensor, or a data signal comprising health information according to a menstrual blood color obtained from the color information.

6. The smart menstrual cup of claim 1, wherein the measuring unit comprises a weight sensor, and
the control unit generates a data signal comprising weight information of menstrual blood measured via the weight sensor, or an alarm signal according to whether a weight of the menstrual blood is greater than a predetermined reference weight.

7. The smart menstrual cup of claim 1, wherein the measuring unit is a sensor formed on an inner side of the storage unit where the menstrual blood is received, and
the control unit generates an alarm signal according to whether a level, an amount, or a volume of menstrual blood measured via the measuring unit is greater than a reference value.

8. The smart menstrual cup of claim 1, further comprising a sense signal generator for generating a sense signal capable of being sensed via at least one of five human senses, based on the signal generated in the control unit.

9. The smart menstrual cup of claim 1, wherein, in the storage unit, a circumference of a location spaced apart by a first distance from the open side of the storage unit is greater than a circumference of a location spaced apart by a second distance greater than the first distance from the open side of the storage unit.

10. The smart menstrual cup of claim 1, wherein, in the storage unit, an area of a cross-section at a location spaced apart by a first distance from the open side of the storage unit is greater than an area of a cross-section at a location spaced apart by a second distance greater than the first distance from the open side of the storage unit.

11. A method of measuring menstrual blood by using a smart menstrual cup, the method comprising:
receiving menstrual blood via a storage unit of the smart menstrual cup;
measuring the menstrual blood stored in the storage unit via a measuring unit of the smart menstrual cup; and
generating a signal, based on measurement information from the measuring unit or processing information obtained based on the measurement information, via a control unit of the smart menstrual cup.

12. The method of claim 11, wherein the measuring comprises measuring a color of the menstrual blood by using a color sensor, and
the generating comprises generating a data signal comprising color measurement information of the menstrual blood, or a data signal comprising health information according to a menstrual blood color obtained from the color information.

13. The method of claim 11, wherein the measuring comprises measuring a weight of the menstrual blood by using a weight sensor, and
the generating comprises generating a data signal comprising weight measurement information of the menstrual blood, or an alarm signal according to whether a weight of the menstrual blood is greater than a predetermined reference weight.

14. The method of claim 11, wherein the measuring comprises measuring a level, an amount, or a volume of the menstrual blood by using a sensor located on an inner side of the storage unit, and
the generating comprises generating a data signal comprising measurement information regarding the level, the amount, or the volume of the menstrual blood, or an alarm signal according to whether the amount of the menstrual blood is greater than a reference value.

15. The method of claim 11, further comprising, after the generating, generating a sense signal capable of being sensed via at least one five human senses, based on the signal generated in the control unit, via a sense signal generator of the smart menstrual cup.
